(19) Europäisches Patentamt
European Patent Office
Office européen des brevets

(11) **EP 1 676 558 B1**

(12) **EUROPÄISCHE PATENTSCHRIFT**

(45) Veröffentlichungstag und Bekanntmachung des Hinweises auf die Patenterteilung:
**11.05.2011 Patentblatt 2011/19**

(51) Int Cl.:
***A61K 8/02*** *(2006.01)*

(21) Anmeldenummer: **05026363.1**

(22) Anmeldetag: **02.12.2005**

(54) **Kosmetisches Wattepad**

Cosmetic pad

Tampon cosmétique

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **16.12.2004 DE 102004060623**

(43) Veröffentlichungstag der Anmeldung:
**05.07.2006 Patentblatt 2006/27**

(73) Patentinhaber: **Paul Hartmann AG**
**89522 Heidenheim (DE)**

(72) Erfinder:
• **Römpp, Angela**
**73119 Zell u.A. (DE)**
• **Schmidt, Heike**
**89542 Herbrechtingen (DE)**
• **Mangold, Rainer, Dr.**
**89542 Herbrechtingen (DE)**

(56) Entgegenhaltungen:
**EP-A- 1 106 723     EP-A- 1 310 226**
**WO-A-03/090653     WO-A-2004/007824**
**WO-A-2004/097096**

## Beschreibung

Die Erfindung betrifft ein kosmetisches Wattepad mit wenigstens einer Faservlieslage und einer ersten und einer zweiten Seite und mit zwei verschiedenen Prägestrukturen auf wenigstens einer Seite, gegebenenfalls mit einer zusätzlichen durch Wasserstrahlvernadelung gebildeten Feinrillenstruktur auf einer oder beiden Oberflächen.

Kosmetische Wattepads haben einen vielfältigen Einsatzbereich. So werden Wattepads zur Reinigung der Haut verwendet, wobei die Haut auch von hauteigenen Absonderungen, wie Fett, befreit wird oder es werden zuvor aufgetragene Schichten, wie Schminke öder Masken, abgewischt. Für Reinigungszwecke werden Wattepads oftmals mit einer die Reinigung unterstützenden Flüssigkeit, wie z. B. Wasser, lösungsmittelhaltige Lotionen oder dergleichen, benetzt oder getränkt. Wattepads dienen oftmals auch als Hilfsmittel zum Auftragen von kosmetischen Produkten, wie z. B. Cremes, auf die Haut. Es ist auch denkbar, dass Wattepads auf die Hautoberfläche aufgelegt werden und dann als Verteilungsmittel für kosmetische Extrakte, die auf das Wattepad aufgegeben werden, dienen. Auch für medizinische Anwendungen kommen kosmetische Wattepads in Betracht.

Aus den vorausgehend beschriebenen Verwendungen ergeben sich spezifische Anforderungen an ein Wattepad. Es soll dem Benutzer eine weiche Anfühlung vermitteln, dabei aber dennoch eine gute Reinigungswirkung verbunden mit einer hohen Stabilität und Festigkeit aufweisen. Darüber hinaus soll eine hinreichende Saugfähigkeit und Flüssigkeitshaltefähigkeit gegeben sein.

Aus EP 1 106 723 A1 ist es bereits bekannt, eine oder beide Seiten eines Wattepads mit einer Wasserstrahlvernadelung zu versehen, die auf den Oberflächen ein feines Rillenmuster bildet und eine Einbindung der Fasern an der Oberfläche bewirkt, so dass das Wattepad weniger zum Ausflusen neigt.

Daneben sind Wattepads mit durchgehend geprägten Oberflächen bekannt, wobei unter "geprägt" hierbei Vertiefungen mit einer Tiefe von größer 0,2 mm, insbesondere von wenigstens 0,25 mm, insbesondere von wenigstens 0,35 mm verstanden werden, also Vertiefungen, die eine größere Tiefe aufweisen als die dem Fachmann bekannte, durch Wasserstrahlvernadelung erzeugte Feinrillenstruktur mit einer Tiefe von lediglich 0,05 - 0,2 mm. Dabei kann ein bereits vorverfestigtes Vliesmaterial insbesondere mit Hilfe eines Prägekalanders, gegebenenfalls auch unter Einwirkung von Wärme, mit einer Prägestruktur versehen werden. Geprägte Oberflächen lassen sich auch mittels, Wasserstrahlvernadelung, insbesondere unter Anwendung der Siebtrommeltechnologie erzeugen, so wie in EP 1 310 226 A2 beschrieben. EP 1 310 226 A2 offenbart ein kosmetisches Wattepad, dessen beide Seiten mit einer durch Wasserstrahlvernadelung hergestellten Feinrillenstruktur versehen sind und wobei zusätzlich auf wenigstens einer Seite eine der Feinrillenstruktur überlagerte geprägte Struktur von Stegen und Vertiefungen ausgebildet ist. Diese Prägestruktur wird im Zuge der Herstellung des Wattepads vor dem Ausstanzen der einzelnen Pads aus einer Flachmaterialbahn auf diese Flachmaterialbahn aufgebracht, so dass die einzelnen Wattepads dann mit eher zufälliger Orientierung zu der Prägestruktur aus der geprägten Flachmaterialbahn herausgestanzt werden.

Auch EP 0 405 043 B1 offenbart Wattepads mit einem Prägemuster, insbesondere in Form eines Waffelmusters, welches sich über die Ränder erstreckt.

Mit WO 03/090653 wird der Vorschlag unterbreitet, dass ein Wattepad an seinem Umfang über einen in Umfangsrichtung durchgehenden Verdichtungsbereich verfügt, der jedoch in einem radialen Abstand zum Umfangsrand des Wattepads vorgesehen sein soll. Auf diese Weise sollen mehrere Materiallagen des Wattepads miteinander verbunden werden, wobei Fasern, die sich von diesem Verdichtungsbereich nach außen zum Rand hin erstrecken und eine entsprechende Länge aufweisen, nicht steif sondern vielmehr leicht biegbar sind. Das Wattepad soll also mit einer in Umfangsrichtung umlaufenden Randverprägung versehen werden, die nicht unmittelbar den Rand bildet, sondern geringfügig radial innerhalb des Rands verläuft.

Aus EP 1 310 226 A2 sind Wattepads bekannt, die Einsenkungen mit verschiedenen geometrischen Konturen und zwischen den Einsenkungen verlaufende Rippenscharen aufweisen.

Auch WO 03/090653 offenbart bereits Wattepads mit zwei unterschiedlichen Prägestrukturen, nämlich mit einer in Umfangsrichtung verdichteten Rille, wobei mindestens eine der Außenlagen weitere Prägestellen aufweisen kann.

Der vorliegenden Erfindung liegt die Aufgabe zugrunde, ein Wattepad der eingangs genannten Art dahingehend zu verbessern dass es einerseits einen Bereich mit verbesserter Abreinigungswirkung bei weicher Anfühlung und dass es andererseits dennoch eine hinreichende Padverfestigung bei der Verwendung des Wattepads durch den Benutzer aufweist.

Diese Aufgabe wird bei einem Wattepad der beschriebenen Art erfindungsgemäß dadurch gelöst, dass eine erste Prägestruktur vollumfänglich (radial außen) von einem äußeren Bereich mit einer von der ersten Prägestruktur verschiedenen zweiten Prägestruktur umgeben ist, wobei die zweite Prägestruktur sich nicht in die erste Prägestruktur hinein erstreckt.

Die erfindungsgemäßen Wattepads haben vorzugsweise eine kreisförmige Form, vorzugsweise sind auch ovale Formen, ebenso quadratische oder rechteckige Formen denkbar. Vorzugsweise haben die kreisförmigen Wattepads einen Durchmesser von 5-7 cm. Ovale Pads umfassen eine Fläche mit den Maßen von vorzugsweise (60 - 80 mm) x (80 - 100 mm). Quadratische Pads haben vorzugsweise eine Seitenlänge zwischen 60 - 80 mm, insbesondere von 75 mm. Rechteckige Pads haben eine Fläche mit Maßen von vorzugsweise (70 - 100 mm) x (90 mm - 120 mm), insbesondere (85 mm - 95 mm) x (105 mm -115 mm). Die Flächengewichte der Wattepads betragen vorteilhafterweise zwischen 70 und 350 g/m², insbesondere zwischen 100 - 300 g/m², ganz insbesondere zwischen 150 - 250 g/m².

Wenn vorstehend von Wattepads die Rede ist, so werden hierunter zwar im allgemeinen absorbierende padförmige Faservliesartikel verstanden, deren Faseranteil aus Baumwollfasern gebildet ist. Es sei aber ausdrücklich darauf hingewiesen, dass auch ein padförmiger Faservliesartikel, dessen Faseranteil aus anderen zellulosischen Fasern gebildet ist, oder der außer zellulosischen Fasern noch Zusätze synthetischer Fasern wie z.B. thermoplastische Fasern beispielsweise aus Polyester, Polyamid, Polyacryl, Polyolefinen, Polyurethan, auch als Mehrkomponentenfasern, insbesondere als Bikomponentenfaser, und vorzugsweise als Mikrofasern mit einer Faserstärke von ≤ 1 dtex, insbesondere Mikrostapelfasern einer Länge von wenigstens 7mm, umfasst, als Wattepad im Sinne der vorstehenden Erfindung und auch im Sinne der Verkehrsgewohnheiten bezeichnet wird. Die erfindungsgemäßen Wattepads mit Zusätzen synthetischer Fasern umfassen insbesondere bis zu 95 Gew-%, insbesondere bis zu 72 Gew-%, weiter insbesondere 15-65 Gew-%, und weiter insbesondere 50-65 Gew-% Baumwollfasern. Wattepads mit wärmeschmelzbare Bindefasern, insbesondere Mehrkomponentenfasern, ganz insbesondere Bikomponentenfasern haben einen gewichtsprozentualen Bindefaseranteil von vorzugsweise 10-20 Gew-%, insbesondere 12-18 Gew-%, ganz insbesondere 12-15 Gew-%.

Wattepads mit Mikrofasern, inbesondere Mikrostapelfasern, weisen insbesondere zu 40-90 Gew-%, insbesondere 15-85 Gew-%, weiter insbesondere 15-65 Gew.-% und weiter insbesondere 20-30 Gew.-% Mikrofasern in der Faservlieslage auf.

Eine besonders vorteilhafte Zusammensetzung kann dabei 55-70 Gew.-% Baumwollkämmlinge, 20-30 Gew.-% PES-Mikrostapelfasern und 10-25 Gew.-% CO-PES/PES-Bikomponentenfasern umfassen.

Eine weitere beispielhafte vorteilhafte Zusammensetzung umfasst 80-90 Gew-% Baumwolle und 10-20 Gew-% thermoplastische Bikomponentenfasern.

Zumindest grundsätzlich wäre auch die Herstellung eines padförmigen Faservliesartikels denkbar, der zum überwiegenden Teil aus synthetischen Fasern gebildet ist. Solchenfalls müssten aber in ganz erheblichem Maße Hydrophilierungsmittel eingesetzt werden, um zu einem absorbierenden saugfähigen Artikel zu gelangen, weshalb üblicherweise bei der Herstellung von Wattepads zellulosische Fasern und vorzugsweise Baumwollfasern als natürliche hydrophile Fasern eingesetzt werden. Wattepads mit einem hohen, vorzugsweise 100 % betragenden Baumwollfaseranteil werden im Hinblick auf eine weiche Anfühlung und ein gutes Flüssigkeitsaufnahmeverhalten bevorzugt.

Mit der Erfindung wird also vorgeschlagen, dass eine erste Prägestruktur nicht die gesamte Oberfläche der betrachteten Seite des Wattepads überfängt, sondern von einem äußeren Bereich mit einer von der ersten Prägestruktur verschiedenen zweiten Prägestruktur vollumfänglich umgeben ist. Die erste Prägestruktur erstreckt sich nicht bis zum Umfangsrand hin oder in einem Umfangsrandabschnitt, sondern diese erste Prägestruktur ist vollumfänglich, also in Umfangsrichtung durchgehend von einem äußeren Bereich mit einer von der ersten Prägestruktur verschiedenen zweiten Prägestruktur umgeben.

Das erfindungsgemäße Wattepad zeichnet sich einerseits durch eine im Wattepad angeordnete erste Prägestruktur aus, die dem Wattepad eine weiche Anfühlung vermittelt und ebenso eine verbesserte Reinigungsleistung ermöglicht. Gleichzeitig wird durch den auf zumindest einer Seite des Wattepads vorgesehenen und die erste Prägestruktur voll umfänglich umgebenden äußeren Bereich, der seinerseits eine von der ersten Prägestruktur verschiedene zweite Prägestruktur aufweist, ein hinreichender Zusammenhalt und eine hinreichende Festigkeit des Wattepads am Randbereich erzielt, die eine verbesserte Griffigkeit des Wattepads z.B. beim Entnehmen aus der Packung ermöglicht.

In Weiterführung des erfindungsgemäßen Wattepads beträgt die radiale Erstreckung des äußeren, die zweite Prägestruktur umfassenden Bereiches ausgehend vom Rand zumindest abschnittsweise wenigstens 5 mm.

Wenn vorausgehend davon die Rede ist, dass die radiale Erstreckung des äußeren, die zweite Prägestruktur umfassenden Bereiches ausgehend vom Rand zumindest abschnittsweise wenigstens 5 mm beträgt, so sei hierdurch zum Ausdruck gebracht, dass dieser äußere Bereich nicht über den gesamten Umfang des Wattepads also 360°, diese radiale Tiefe oder Erstreckung von wenigstens 5 mm aufweisen muss. Es erweist sich als vorteilhaft, wenn dieser äußere Bereich über einen Abschnitt von wenigstens 180° in Umfangsrichtung, insbesondere von wenigstens 270°, insbesondere von wenigstens 280°, insbesondere von wenigstens 290°, insbesondere von wenigstens 300°, insbesondere von wenigstens 310° die radiale Erstreckung vom Rand nach innen von wenigstens 5 mm aufweist. Der jeweilige erwähnte Abschnitt kann sich auch aus einzelnen in Umfangsrichtung alternierenden Teilabschnitten zusammensetzen.

In Weiterbildung der Erfindung erweist es sich als vorteilhaft, wenn die radiale Erstreckung des äußeren, die zweite Prägestruktur umfassenden Bereichs ausgehend vom Rand nach radial innen über die gesamte Umfangsrichtung wenigstens 3 mm, insbesondere wenigstens 4 mm und weiter insbesondere wenigstens 5 mm beträgt.

Nach einer weiteren vorteilhaften Variante beträgt die radiale Erstreckung ausgehend vom Rand zumindest abschnittsweise wenigstens 8 mm, wobei vorteilhafterweise dieser äußere Bereich über einen Abschnitt von wenigstens 180° in Umfangsrichtung, insbesondere von wenigstens 190°, insbesondere von wenigstens 200° eine radiale Erstreckung vom Rand nach innen von wenigstens 8 mm aufweist.

In noch weiterer Ausbildung der Erfindung beträgt die radiale Erstreckung des äußeren, eine zweite Prägestruktur umfassenden Bereichs ausgehend vom Rand nach innen zumindest abschnittsweise wenigstens 10 mm, wobei dieser äußere Bereich vorzugsweise über einen Abschnitt von wenigstens 120° in Umfangsrichtung, insbesondere von wenigstens 150°, insbesondere von wenigstens 160° eine radiale Erstreckung vom Rand nach innen von wenigstens 10 mm aufweist.

Mit der vorliegenden Erfindung wurde auch erkannt, dass der gesamte prozentuale Flächenanteil der Prägelinien oder Prägestellen durchaus nicht sehr hoch zu sein braucht und dass - was im folgenden noch erläutert werden wird - Prägestrukturen entgegen weit verbreiteter Ansicht sich eher nicht dazu eignen, Make-up oder dergleichen aufzunehmen, sondern eher die Reinigungswirkung verschlechtern. Bei einer vorteilhaften Ausführungsform des erfindungsgemäßen Wattepads umfasst die erste Prägestruktur eine Fläche der Prägelinien oder Prägestellen, bezogen auf die Padgesamtfläche, von nur 1% bis 8%, insbesondere von 2% bis 7% und weiter insbesondere von 3 % bis 6% der Oberfläche einer betreffenden Seite des Wattepads.

Die erste Prägestruktur kann an sich beliebig ausgebildet sein; sie kann gepunktet, gestrichelt oder auch als kontinuierliche Prägelinien ausgebildet sein. Sie kann mindestens eine geschlossene Struktur, etwa dem Umfang einer Sternstruktur oder einer Blumenstruktur folgend ausgebildet sein, oder sie kann einen in Umfangsrichtung unterbrochenen Abschnitt aufweisen. Die erste Prägestruktur kann dabei eine visuell oder taktil wahrnehmbare Information vermitteln, insbesondere ein Dekorationselement, weiter insbesondere einen Schriftzug darstellen. Als vorteilhaft haben sich für die Darstellung der ersten Prägestruktur Prägelinien einer Breite von 0,24 mm - 0,7 mm und insbesondere einer Länge von 0,24 mm - 130 mm, insbesondere einer Länge von 0,24 mm - 100 mm, insbesondere einer Länge von 0,24 mm - 80 mm, insbesondere von 0,24 mm - 60 mm, insbesondere von 0,24 mm - 50 mm, insbesondere von 0,24 mm - 40 mm, insbesondere von 0,24 mm - 30 mm, insbesondere von 1,5 mm - 20 mm und ganz insbesondere von 2 mm - 10 mm erwiesen. Bei einer loch- oder punktförmigen, insbesondere kreisförmigen Prägung haben sich Abmessungen von 0,24 mm - 0,7 mm als vorteilhaft erwiesen.

Die erste Prägestruktur kann in Form einer Vertiefung mit einer Tiefe von größer 0,2 mm, insbesondere von wenigstens 0,25 mm, weiter insbesondere von wenigstens 0,35 mm in einer Seite des Pads ausgebildet sein, oder sie kann durchgeprägt sein, so dass eine Öffnung in dem Wattepad gebildet wird. Die erste Prägestruktur kann dabei aus Prägungselementen von unterschiedlicher Tiefe zusammengesetzt sein. Insbesondere können sowohl Vertiefungen als auch Durchprägungen vorgesehen sein.

In weiterer Ausbildung des Erfindungsgedankens beträgt der Flächenanteil des äußeren Bereiches umfassend die zweite Prägestruktur, welcher die erste Prägestruktur in Umfangsrichtung umgibt, wenigstens 30 %, insbesondere wenigstens 35 %, insbesondere wenigstens 40 %, insbesondere wenigstens 45 % , weiter insbesondere wenigstens 48 %, insbesondere wenigstens 50 % und weiter insbesondere 50 % bis 65 % der Oberfläche der betreffenden Seite.

Die zweite Prägestruktur ist Teil des äußeren Bereiches, der die erste Prägestruktur vollumfänglich umgibt. Diese zweite Prägestruktur kann an sich beliebig ausgebildet sein; sie kann gepunktet, gestrichelt oder auch als kontinuierliche Prägelinien, in geradliniger, welliger oder zickzack - Form ausgebildet sein. Vorzugsweise zeigt die zweite Prägestruktur in sich zweidimensional kontinuierliche Vertiefungen. Insbesondere bevorzugt ist die zweite Prägestruktur dabei als gitterförmige, oder wabenförmige, oder fächerförmige oder halbkreisförmige Vertiefungen ausgebildet. Die Vertiefungen der zweiten Prägestruktur weisen eine Tiefe von wenigstens 0,2 mm, insbesondere von wenigstens 0,25 mm, weiter insbesondere von wenigstens 0,35 mm in einer Seite des Pads auf. Die Prägelinien weisen insbesondere eine Breite von 0,24 mm - 0,7 mm auf.

In noch weiterer Ausbildung der Erfindung beträgt der Prägeflächenanteil, das heißt der Flächenanteil der Prägelinien oder Prägestellen, des äußeren Bereiches mit einer zweiten Prägestruktur, 3% bis 11%, insbesondere 5% bis 10% und weiter insbesondere 7,0% bis 9,5% der Oberfläche einer betreffenden Seite des Wattepads.

In bevorzugter Ausführungsform umfasst der äußere Bereich, der die erste Prägestruktur vollumfänglich umgibt, zusätzlich zu der zweiten Prägestruktur auch einen ungeprägten Bereich. Die räumliche Anordnung dieses ungeprägten Bereiches und der zweiten Prägestruktur innerhalb des äußeren Bereiches kann beliebig gestaltet sein. Dieser ungeprägte Bereich ist dabei vorzugsweise zwischen der ersten Prägestruktur und der zweiten Prägestruktur angeordnet. Dieser ungeprägte Bereich kann dabei einen Anteil von 7 % - 18 %, bevorzugt von 8 % - 17 % bezogen auf die Gesamtfläche einer Oberseite des Wattepads aufweisen.

In weiterer Ausbildung der Erfindung weisen erste und zweite Prägestruktur einen unterschiedlichen Prägeflächenanteil auf. Insbesondere ist der Prägeflächenanteil der zweiten Prägestruktur größer als der Prägeflächenanteil der ersten Prägestruktur.

Die erste Prägestruktur weist einen Anteil an verprägter Fläche innerhalb der von erster Prägestruktur eingenommenen Fläche von vorzugsweise 5,5 % bis 14,0 %, bevorzugt von 7,5 % bis 12,5 %, insbesondere bevorzugt von 9,5% bis 11,5 % auf.

Die zweite Prägestruktur weist einen Anteil an verprägter Fläche innerhalb der von der zweiten Prägestruktur eingenommenen Fläche von vorzugsweise wenigstens 12 %, bevorzugt wenigstens 14 %, weiter bevorzugt wenigstens 16 %, insbesondere bevorzugt wenigstens 18 % bis höchstens 22 %, weiterhin bevorzugt 19 % bis 21 % auf.

Die durch die erste Prägestruktur und zweite Prägestruktur beschriebenen Flächen des Wattepads weisen vorteilhafterweise unterschiedlichen Dicken auf. An der durch die erste Prägestruktur beschriebenen Fläche ist das Wattepad dicker als an der durch die zweite Prägestruktur beschriebenen Fläche, gemessen bei einem Prüfdruck von 0,5 kPa.

Wir bereits erwähnt handelt es sich bei Festigkeitseigenschaften von Wattepads und bei dem Erfordernis, eine möglichst weiche Anfühlung jedoch unter Beibehaltung einer guten Reinigungswirkung bereitzustellen, um konfliktierehde Zielsetzungen: Testet man die innere Festigkeit von Wattepads, die ein Maß für den Widerstand gegen Delaminieren darstellt, so zeigen sich Wattepads mit bekannten Prägemustern als diesbezüglich sehr stabil, jedoch mit den eingangs erwähnten Nachteilen. Mit der vorliegenden Erfindung wurde festgestellt, dass sich durch die beanspruchte Ausbildung des Wattepads dennoch eine für die Gebrauchseigenschaften hinreichende innere Festigkeit von wenigstens 0,20 N/25,5cm$^2$, insbesondere von wenigstens 0,30 N/25,5cm$^2$, insbesondere von wenigstens 0,35 N/25,5cm$^2$, insbesondere von wenigstens 0,40 N/25,5cm$^2$ und insbesondere bis 1,5 N/25,5cm$^2$ und weiter insbesondere bis 1,0 N/25,5cm$^2$ erreichen lässt.

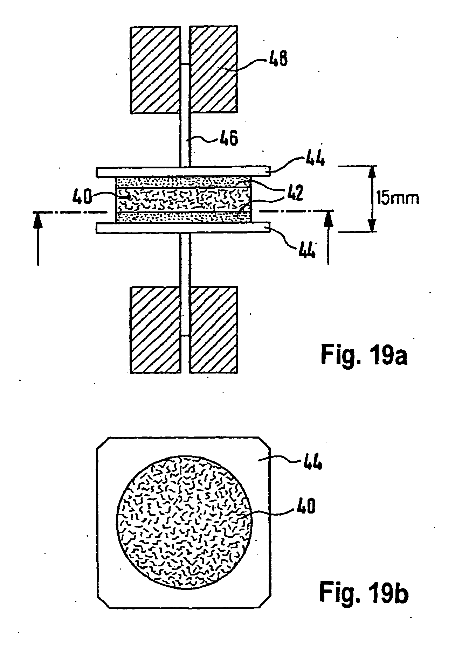

Es erweist sich des weiteren als vorteilhaft, dass bei dem erfindungsgemäßen Wattepad bei der Bestimmung der inneren Festigkeit die maximale Dehnungskraft erst bei oberhalb von 20 % Dehnung, insbesondere bei oberhalb von 25 % Dehnung und weiter insbesondere zwischen 30 % und 50 % Dehnung des Materials des Wattepads bezogen auf eine Einspannlänge von 15 mm, die aus Figur 19a ersehen werden kann, auftritt. Dies erweist sich bei einer beanspruchenden Benutzung des Wattepads als vorteilhaft, weil es als komfortabel und angenehm im Gebrauch empfunden wird.

Die Reißfestigkeit eines erfindungsgemäßen Wattepads im trockenen Zustand beträgt in Längsrichtung und vorzugsweise auch in Querrichtung 3 bis 20 N/25 mm, insbesondere bevorzugt 5 bis 10 N/25 mm wobei zur Bestimmung der Reißfestigkeit von aus dem Wattepad ausgestanzten Prüflingen einer Abmessung von 25 mm x 30 mm ausgegangen wurde. Die vorstehend genannten vorteilhaften Werte beziehen sich auf ein zu 100% aus Baumwollfasern gebildetes Wattepad mit einer Dicke von 2,3 bis 3,3 mm (ermittelt bei einem Prüfdruck von 0,5 kPa) Die Masse eines solchen Wattepads mit ca. 5,7 cm Durchmesser liegt bei ca. 0,5 bis 0,6 g.

Test der inneren Festigkeit flächenhafter Faservliesartikel:

Hierfür kann eine Zugprüfmaschine nach DIN 51221, Klasse 1 sowie zwei Hilfsbleche und doppelseitiges Klebeband verwendet werden. Eine flächenhafte kreisscheibenförmige Probe von 57 mm Durchmesser wird mittels des doppelseitigen Klebebands zwischen ein oberes und ein unteres Hilfsblech geklebt, von dem senkrecht ein einspannbarer Haltesteg vorsteht (T-Form). Sollten nur kleinere Pads zur Verfügung stehen oder getestet werden können, so kann auch mit einer flächenhaften kreisscheibenförmigen Probe von 28,5 mm Durchmesser gearbeitet werden, wobei dies dann bei den Ergebnissen anzugeben wäre. Solchenfalls ist das Ergebnis auf eine Prüffläche von 25.5 cm$^2$ rechnerisch zu normieren. Die beiden Hilfsbleche werden in die Zugprüfmaschine eingespannt und dann mit einer Geschwindigkeit von 100 mm/min auseinanderbewegt; währenddessen wird die auftretende Zugkraft gemessen. Es wird dann die Höchstzugkraft ermittelt. Unter der Höchstzugkraft wird diejenige maximale Kraft verstanden, bei der der Zusammenhalt des Wattepads zerstört wird. Wenn zuvor höhere Kraftspitzen im Zuge der Dehnung gemessen werden, so stellen diese die Höchstzugkraft im Sinne dieser Prüfung dar.

Figuren 19 a) und b) zeigen schematisch den Versuchsaufbau. Man erkennt den kreisscheibenförmigen Prüfling in Form eines Wattepads 40, sowie daran angrenzend jeweils das doppelseitige Klebeband 42 und die Hilfsbleche 44, die mit ihrem abstehenden Steg 46 in Klemmaufnahmen 48 der Zugprüfmaschine eingespannt sind. Auch die Einspannlänge von 15 mm ist ersichtlich.

Bei dem doppelseitigen Klebeband handelt es sich um ein Klebeband der Firma 3M (Tape 410) mit Naturkautschuk als Klebebeschichtung und einer vorzugsweise definierten. Klebekraft von 19,3 +/- 2,2 N/25 mm nach dem Deutschen Arzneibuch von 1996 (dort beschriebene Abziehmethode).

Zur Probenpräparation wird ein der Größe des Wattepads entsprechender Abschnitt des vorstehend erwähnten Doppelklebebands jeweils oben und unten auf die Wattevliesschicht der zu testenden Wattepads aufgeklebt. Der so erhaltene Verbund wird (nach Abziehen der äußeren Deckschichten des Klebebands) zwischen den zwei Hilfsblechen angeordnet und zentrisch positioniert. Sodann werden die Hilfsbleche mittels eines Gewichtes einer Masse von 1 kg 1 min lang beschwert, so dass der Verbund aus Wattepad und Doppelklebeband und Hilfsblechen innig miteinander verbunden wird. Dieser Verbund wird dann in die Zugprüfmaschine nach DIN 51221 eingespannt, und mit der erwähnten Geschwindigkeit von 100 mm/min werden die Klemmen auseinandergezogen und dabei die Zugkraft ermittelt. Aus wenigstens fünf Einzelmessungen wird der Mittelwert gebildet und in N angegeben.

Für die reproduzierbare Durchführung von Messungen wird die Klebekraft des doppelseitigen Klebebands nach der erwähnten Abziehmethode des Deutschen Arzneibuchs 1996 angegeben bzw. standardisiert. Hierfür wird diejenige Kraft gemessen, die erforderlich ist, um Klebebänder (z.B. Pflaster) von einem ebenen Untergrund im Winkel von 180° mit einer konstanten Geschwindigkeit abzuziehen. Wiederum wird eine Zugprüfmaschine nach DIN 51221 Klasse 1 hierfür verwandt. Es kommen Platten aus rostfreiem Stahl, 150 x 50 x 2 mm mechanisch poliert und in Längsrichtung geraut zum Einsatz.

Die Prüfung wird bei 23°C und 50 % relativer Luftfeuchte durchgeführt. Zuvor sind die Proben 24 Stunden lang unter diesen Standardbedingungen zu lagern. Die Stahlplatten werden vor Beginn mit einem Toluol getränkten Wattebausch gereinigt, dann werden sie in einem geeigneten Behältnis mit den Dämpfen von siedendem Toluol in Kontakt gebracht, ohne dass sie die Flüssigkeit jedoch direkt berühren. Die so erhaltenen Dämpfe streichen 5 min lang entlang der Plattenoberflächen. Danach werden die Platten 30 min lang im Standardklima erkalten gelassen.

Es werden dann Streifen von 400 mm Länge und vorgegebener Breite von der Rolle von 12,5 oder 25 mm, zugeschnitten und auf die gereinigten Metallplatten derart aufgebracht, dass Lufteinschlüsse vermieden werden. Mittels eines "Tape-Applicators" wird unter einem Druck von 20 N/cm Probenbreite der Klebebandstreifen angerollt (wobei das rückseitige Deckpapier des Klebebands noch nicht entfernt wurde). Nach 10 min Wartezeit erfolgt dann die Messung.

Zur Messung wird das obere freie Ende des Probenstreifens zurückgeschlagen und etwa 25 mm vom oberen Ende der Stahlplatte abgezogen. Dieses Ende der Stahlplatte wird in die obere Klemme der Zugprüfmaschine eingespannt und das zurückgeschlagene Ende des Probenstreifens wird in die untere Klemme der Zugprüfmaschine eingespannt. Der Abzugswinkel beträgt somit 180°, wobei darauf zu achten ist, dass die Probenrückseiten parallel zueinander sind, nicht jedoch aneinander reiben. Die Zugprüfmaschine wird auf eine Abzugsgeschwindigkeit von 300 +/- 30 mm/min eingestellt.

Zur Ermittlung der Klebekraft ist der Kraftverlauf zu ermitteln und aufzuzeichnen. Aus den erhaltenen Kraftspitzen ist dann die mittlere Klebekraft nach einem der nachfolgend beschriebenen Verfahren A) bis C) auszuwerten.

Bei abweichenden Kurvenverläufen ist gegebenenfalls nach den weiter unten beschriebenen Verfahren A oder B auszuwerten. In diesen Fällen ist das Ausw erteverfahren bei der Resultatsangabe mit anzugeben.

Auswerteverfahren C:

Dieses Verfahren ist anzuwenden, wenn das Diagramm mehr als 20 deutlich erkennbare Kraftspitzen aufweist.

Voraussetzung ist dabei, dass die innerhalb des Diagramms vorkommenden Schwankungen nicht periodisch auftreten. Ist das der Fall, ist das Auswerteverfahren B anzuwenden.

Ausgehend von der Mitte jener Diagrammlänge 1, die von der ersten Kraftspitze bis zum Abriss reicht, sind vier senkrechte Linien in gleichen Abständen von 1/10 dieser Diagrammlänge nach beiden Seiten einzureichen.

Diese Abstände sind auf ganze Millimeter aufzurunden. Die neun Spitzenwerte, die diesen Linien am nächsten liegen, sind zur Bestimmung der Klebkraft heranzuziehen.

Einzelne extrem aus dem Kurvenverlauf herausragende Spitzenwerte werden bei der Auswertung nicht mit berücksichtigt.

Das Ergebnis ist als Mittelwert von mindestens fünf Prüfungen in N/25 mm auf eine Nachkommastelle gerundet anzugeben.

Die Klebekraft wird wie folgt berechnet:

$$F = \frac{\sum_{i=1}^{n} F_i}{n}$$

$F_i$ = Kraftspitzen $F_1, F_2 .... F_n$
n = Anzahl der ausgewerteten Kraftspitzen

Die Auswertung kann auch mit einem geeigneten PC-Programm erfolgen.

Auswerteverfahren A:

Dieses Verfahren ist anzuwenden, wenn das Diagramm bis fünf deutlich unterscheidbare Kraftspitzen aufweist. Der Mittelwert aus den Werten dieser Kraftspitzen ist zu bestimmen.

In dem Fall, dass im Diagramm nur eine Kraftspitze auftritt, ist der entsprechende Wert als "Mittelwert" zu betrachten.

Auswerteverfahren B:

Dieses Verfahren ist anzuwenden, wenn sechs bis zehn deutlich unterscheidbare Kraftspitzen im Diagramm erscheinen.

Die Spitzenwerte der mittleren 80 % jenes Diagrammbereichs, das mit der ersten Kraftspitze beginnt und mit dem Abriss endet, werden zur Bestimmung der Klebkraft herangezogen.

Die vorstehende Beschreibung der Klebekraft dient, wie bereits erwähnt dazu, standardisierte reproduzierbare Bedingungen für das bei dem vorstehend beschriebenen Test der inneren Festigkeit zu verwendende Klebeband zu schaffen.

Reißfestigkeit in Längs- und Querrichtung:

Die vorerwähnte Reißfestigkeit kann unter Verwendung einer genormten Zugprüfmaschine nach DIN 51221 nach der folgenden Prüfmethode ermittelt werden: Es werden aus zu prüfenden Wattepads, und zwar vorzugsweise aus einem mittleren Bereich der Wattepads Proben einer Einspannbreite von 25 mm und einer Einspannlänge von 30 mm herausgeschnitten, vorzugsweise herausgestanzt. Diese Proben werden in Klemmaufnahmen der erwähnten Zugprüfmaschine lotrecht eingespannt. Zur Durchführung der Messung werden die Klemmaufnahmen dann mit einer Prüfgeschwindigkeit von 100 mm/min in entgegengesetzten Richtungen, jedoch in Ebenenrichtung der eingespannten Probe auseinanderbewegt, und dabei wird die durch die Klemmen auf die Probe ausgeübte und in Ebenenrichtung wirkende Zugkraft ständig gemessen. Unter der Höchstzugkraft wird diejenige maximale Kraft verstanden, bei der das Wattepad zerreißt. Wenn zuvor höhere Kraftspitzen im Zuge der Dehnung gemessen werden, so stellen diese die Höchstzugkraft im Sinne dieser Prüfung dar. Man kann vorteilhafterweise bei Messungen der Längs- und der Querrichtung, welche üblicherweise der Maschinenrichtung bzw. der Richtung quer hierzu entspricht, mehrere, insbesondere fünf, Einzelmessungen vornehmen und deren Mittelwert berechnen.

Wie eingangs bereits angedeutet wurde mit der vorliegenden Erfindung auch festgestellt, dass durch Prägung erhaltene Vertiefungen in einem Wattepad die Reinigungswirkung, also die Fähigkeit, Make-up oder Hautreste aufzunehmen, eher nachteilig beeinflussen. Dies wurde festgestellt, in dem Make-up auf einen Streifen von Ziegenglattleder aufgetragen wurde. Nach einer Antrocknung von 2 bis 2,5 Stunden wird über die mit Make-up versehene Stelle ein zuvor befeuchtetes Wattepad mit 5,7 cm Durchmesser, welches mittels doppelseitigen Klebebands mit einem Gewicht von 1 kg belastet ist, über die "geschminkte" Stelle des Ziegenglattleders unter Verwendung einer Zugprüfmaschine nach DIN 51221 mit einer Geschwindigkeit von 200 mm/Minute gezogen. Pro Wattepad werden 5 Abschminkversuche durchgeführt. Es kann dann die auf dem Ziegenleder verbleibende Menge an Make-up entweder visuell oder mittels eines Farbmessgeräts ermittelt werden. Man erkennt, dass Wattepads mit einem sehr großen Anteil von durch Prägung hergestellten Vertiefungen eine schlechtere Abreinigungswirkung haben. Betrachtet man das Wattepad danach, so erscheinen die durch Prägung gebildeten punkt- oder linienförmigen Vertiefungen deutlich heller, weil sich darin - entgegen weit verbreiteter Ansicht - kein Make-up angesammelt hat.

In Weiterbildung der Erfindung kann das Wattepad ein- oder beidseitig eine durchgehende, das heißt auch eine sich in den Rand erstreckende durch Wasserstrahlvernadelung gebildete Feinrillenstruktur von 0,05 - 0,2 mm Tiefe aufweisen.

Weitere Merkmale, Einzelheiten und Vorteile der Erfindung ergeben sich aus den beigefügten Patentansprüchen sowie der zeichnerischen Darstellung und nachfolgenden Beschreibung bevorzugter Ausführungsformen des erfindungsgemäßen Wattepads. In der Zeichnung zeigen:

Figuren 1 bis 5 Draufsichten auf Wattepads nach der Erfindung mit erster und zweiter Prägestruktur;

Figuren 6 bis 14 Draufsichten erfindungsgemäßer Wattepads mit unterschiedlichen Motiven für die erste Prägestruktur;

Figur 15 Wattepad mit der ersten Prägestruktur nach Figur 7 mit eingezeichneten Kreislinien, welche eine Erstreckung des die erste Prägestruktur vollumfänglich umgebenden äußeren Bereichs ausgehend vom Rand von wenigstens 5 mm bzw. von wenigstens 8 mm verdeutlichen;

Figur 16 Wattepad mit der ersten Prägestruktur nach Figur 6 mit eingezeichneter Kreislinie, welche eine Erstreckung des die erste Prägestruktur vollumfänglich umgebenden äußeren Bereichs ausgehend vom Rand von wenigstens 10 mm verdeutlicht.

Figur 17 ein ovales Wattepad mit der ersten Prägestruktur nach Figur 8 mit eingezeichneten konzentrischen Linien, welche eine Erstreckung des die erste Prägestruktur vollumfänglich umgebenden äußeren Bereichs ausgehend vom Rand von 5 mm bzw. von wenigstens 10 mm verdeutlichen.

Figur 18 ein rechteckiges Wattepad mit der ersten Prägestruktur nach Figur 9 mit eingezeichneten zum Rand parallelen Linien, welche eine Erstreckung des die erste Prägestruktur vollumfänglich umgebenden äußeren Bereiches ausgehend vom Rand von wenigstens 5 mm bzw. von wenigstens 10 mm verdeutlichen.

Figur 19 a, b eine schematische Darstellung des Testaufbaus für die Bestimmung der inneren Festigkeit.

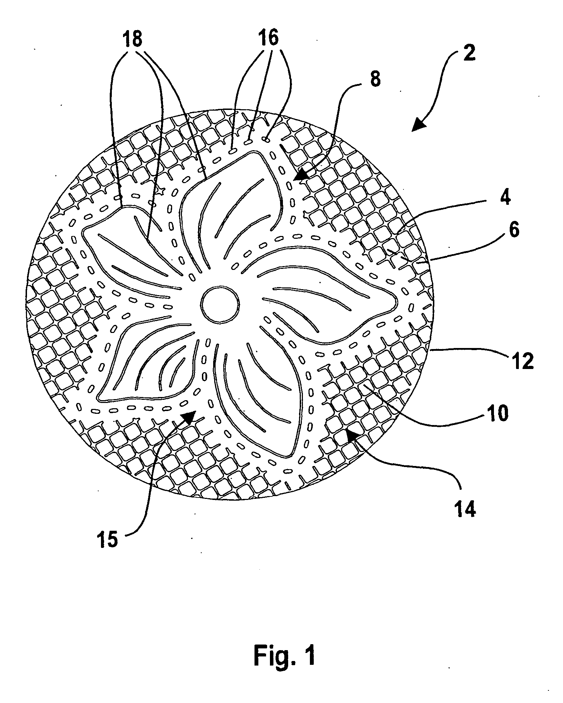

Die Figuren 1 bis 5 zeigen verschiedene Ausführungsformen eines erfindungsgemäßen Wattepads 2. Vorzugsweise haben die Wattepads einen Durchmesser von 5-7 cm. Auch ovale Formen sind denkbar, ebenso quadratische oder rechteckige Formen. Die Wattepads können zu 100% aus Baumwollfasern bestehen oder Zusätze von thermoplastischen Fasern, insbesondere Mehrkomponentenfasern und/oder Mikrofasern umfassen. Die Flächengewichte der Wattepads betragen vorteilhafterweise zwischen 70 und 350 g/m², insbesondere zwischen 100 - 300 g/m², ganz insbesondere zwischen 150 - 250 g/m².

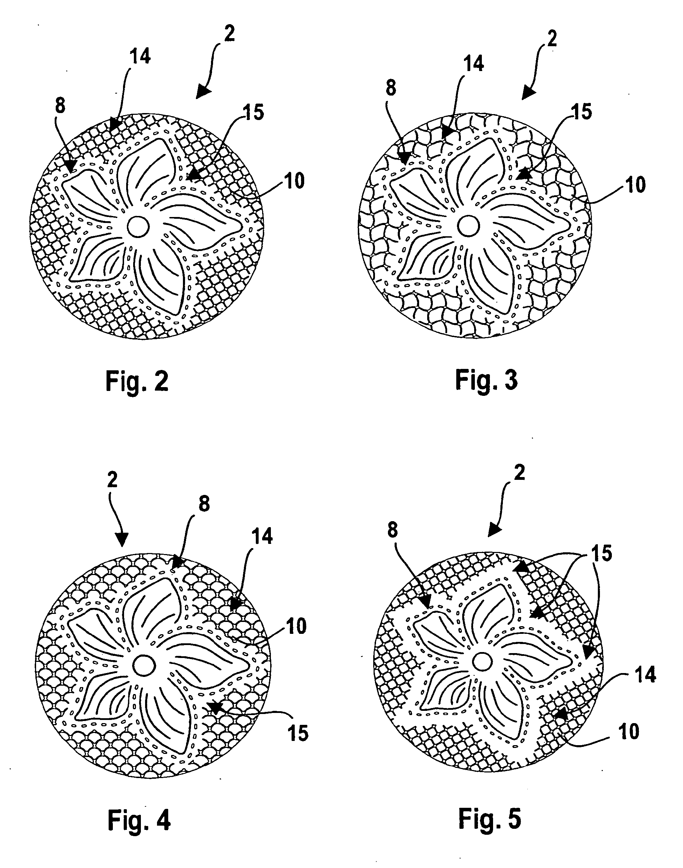

Die in Figuren 2 bis 5 maßstabsgetreu abgebildeten Wattepads haben einen Durchmesser von 5,7 cm.

Anhand der Figur 1 wird das jeweilige Wattepad 2 erläutert: Ein jeweiliges Wattepad 2 weist eine erste Seite 4 und eine zweite der ersten abgewandte Seite 6 auf. Nicht dargestellt ist eine sehr feine nur die Oberflächen erfassende Rillenstruktur, die durch eine Wasserstrahlvernadelung hergestellt ist. Auf der ersten Seite 4 ist zusätzlich eine erste Prägestruktur 8 ausgebildet. Eine jeweilige erste Prägestruktur 8 ist vollumfänglich von einem äußeren Bereich 10 umgeben, der also in Umfangsrichtung durchgehend geschlossen ist. Somit erstreckt sich die erste Prägestruktur 8 an keiner Stelle bis zum Rand 12, also bis zu der äußeren Begrenzung des Wattepads 2, sondern hält an jeder Stelle einen noch näher zu beschreibenden Abstand vom Rand 12 ein. Dieser, die erste Prägestruktur 8 umgebende äußere Bereich 10 weist seinerseits eine zweite Prägestruktur 14 auf. Im dargestellten Fall ist zwischen erster Prägestruktur 8 und zweiter Prägestruktur 14 noch ein unverprägter Bereich 15 vorgesehen.

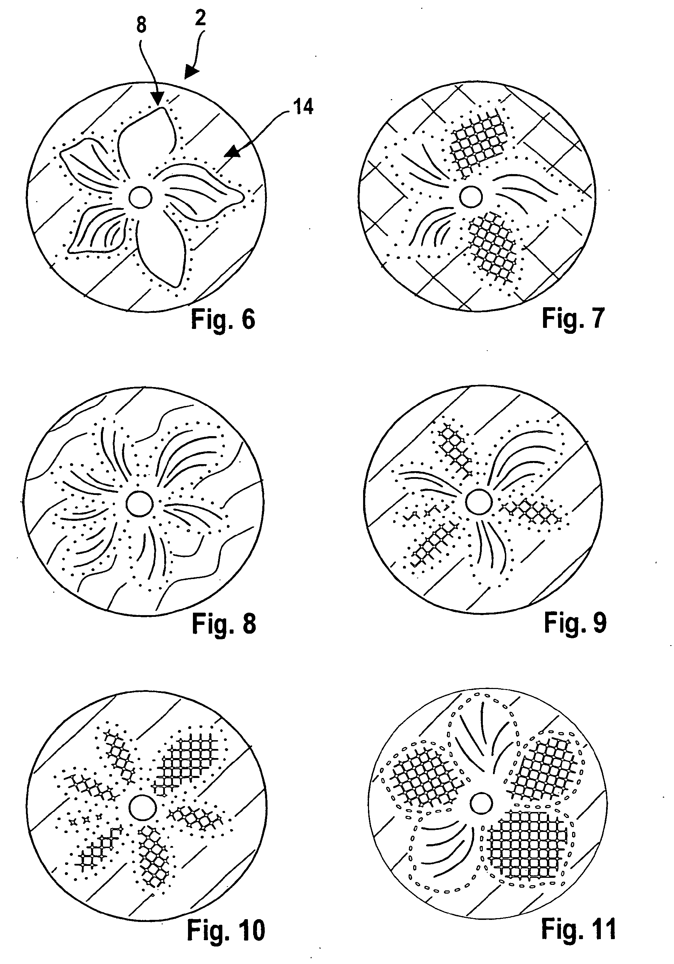

Die erste Prägestruktur 8 weist bei allen Figuren eine äußere Umfangskontur auf, die durch eine Vielzahl von aufeinanderfolgend vorgesehenen ovalen bzw. kreisrunden Prägepunkten 16 gebildet ist. Die Prägepunkte haben eine Prägetiefe von wenigstens 0,25 mm. Innerhalb dieser Umfangskontur sind Prägelinien 18 mit einer Tiefe der Prägung von wenigstens 0,25 mm ausgebildet, die zum Teil der Umfangskontur folgen, zum Teil in etwa in radialer Richtung erstreckt sind. Sie bilden vorliegend ein Motiv. Die Stegbreite der Prägelinien 18 beträgt 0,24 mm. Die Prägepunkte 16 haben eine Dimension von 0,24 mm x 1,0 mm (in den Figuren 2 bis 5) bzw. einen Durchmesser von 0,24 mm (in den Figuren 6 bis 13).

Der Anteil der punktförmig oder linienförmig geprägten Fläche der ersten Prägestruktur 8 an der Gesamtfläche der ersten Seite des Pads 2 beträgt bei dem Ausführungsbeispiel der Figuren 2 bis 4 5,47 %. Beim Ausführungsbeispiel der Figur 5 beträgt dieser Anteil 4,92 %.

Der Flächenanteil des äußeren Bereiches 10 außerhalb der Umfangskontur der ersten Prägestruktur 8, die durch die Prägepunkte 16 gebildet ist, beträgt bei dem Wattepad nach Figuren 2 bis 4 51,9 %, in dem Wattepad nach Figur 5 61,1 % der Gesamtfläche der ersten Seite des Pads 2.

Der äußere Bereich 10 bei den Wattepads gemäß Figur 2 bis 5 weist neben der zweiten Prägestruktur 14 einen ungeprägten Bereich 15 auf, der zwischen der ersten Prägestruktur 8 und der zweiten Prägestruktur 14 angeordnet ist. In den Ausführungsbeispielen gemäß Figuren 2 bis 4 beträgt der Flächenanteil dieses ungeprägten Bereiches 15 8,7 % der Gesamtfläche der ersten Seite 4 des Pads 2. In dem Ausführungsbeispiel gemäß Figur 5 beträgt der ungeprägte Bereich 15 17,9 % der gesamten Oberfläche der ersten Seite 4.

Der Flächenanteil der geprägten Fläche der zweiten Prägestruktur 14 an der Gesamtfläche der ersten Seite des Pads 2 beträgt bei den Ausführungsbeispielen der Figuren 2, 3, 4, und 5 in dieser Reihenfolge 9,09%, 6,26%, 8,46 % bzw. 9,09%. In den Figuren 2 bis 5 sind Ausführungsbeispiele für die zweite Prägestruktur 14 innerhalb des äußeren Bereiches 10 dargestellt. In Figur 2 umfasst der äußere Bereich 10 eine zweite Prägestruktur 14 in der Ausführung einer Gitterstruktur.

Die Gitterstruktur hat eine Prägetiefe von wenigstens 0,25 mm. Die Breite der geprägten Linien in der Gitterstruktur beträgt 0,24 mm; die Linien sind dabei 2,0 mm beabstandet.

In Figur 3 umfasst der äußere Bereich 10 eine zweite Prägestruktur 14 in der Ausführung einer Wabenstruktur. Die Wabenstruktur hat eine Prägetiefe von wenigstens 0,25 mm. Die Breite der geprägten Linien in der Gitterstruktur beträgt 0,24 mm; die Linien sind dabei 3,26 mm beabstandet.

In Figur 4 umfasst der äußere Bereich 10 eine zweite Prägestruktur 14 in der Ausführung einer Fächerstruktur. Die Fächerstruktur hat eine Prägetiefe von wenigstens 0,25 mm. Die Breite der geprägten Linien in der Gitterstruktur beträgt 0,24 mm.

In Figur 5 besteht der äußere Bereich 10 aus der zweiten Prägestruktur 14 und dem an die erste Prägestruktur 8 angrenzenden, deutlich erkennbaren ungeprägten Bereich 15. Die zweite Prägestruktur 14 ist in der Ausführung einer Gitterstruktur wie in Figur 2 geprägt.

Die Prägeflächenanteile der zweiten Prägestruktur 14 bezogen auf die von der zweiten Prägestruktur eingenommenen Flächen betragen bei den Wattepads gemäß den Figuren 2, 3, 4 und 5 in dieser Reihenfolge 21,04%, 14,49%, 19,58 % bzw. 21,04 %.

Im Vergleich dazu betragen die Prägeflächenanteile der ersten Prägestruktur 8 bezogen auf die von der ersten Prägestruktur eingenommenen Flächen bei den Wattepads gemäß den Figuren 2 bis 4 jeweils 11,37 %, in dem Wattepad gemäß Figur 5 beträgt dieser Anteil 12,64%.

Die Figuren 6 bis 12 zeigen schematisch die erste Prägestruktur 8 in verschiedenen Ausführungen. Das Wattepad nach dieser Erfindung umfasst neben dieser ersten Prägestruktur 8 eine zweite Prägestruktur 14, die jeweils lediglich schematisch angedeutet ist.

Die Prägeflächenanteile der jeweiligen ersten Prägestruktur 8 bezogen auf die Gesamtfläche der ersten Seite des Pads 2 betragen in den Ausführungen der Figuren 6, 7, 8, 9, 10, 11 und 12 in dieser Reihenfolge 3,68%, 4,59 %, 3,20 %, 4,07 %, 5,11 %, 10,79 % bzw. 3,22 %.

Die Prägeflächenanteile der jeweiligen ersten Prägestruktur 8 bezogen auf die von der ersten Prägestruktur eingenommenen Flächen betragen in den Ausführungen der Figuren 6, 7, 8, 9, 10, 11 und 12 in dieser Reihenfolge 7,65%, 9,54 %, 7,77%, 9,865, 12,41%, 16,62 % bzw. 5,60 %.

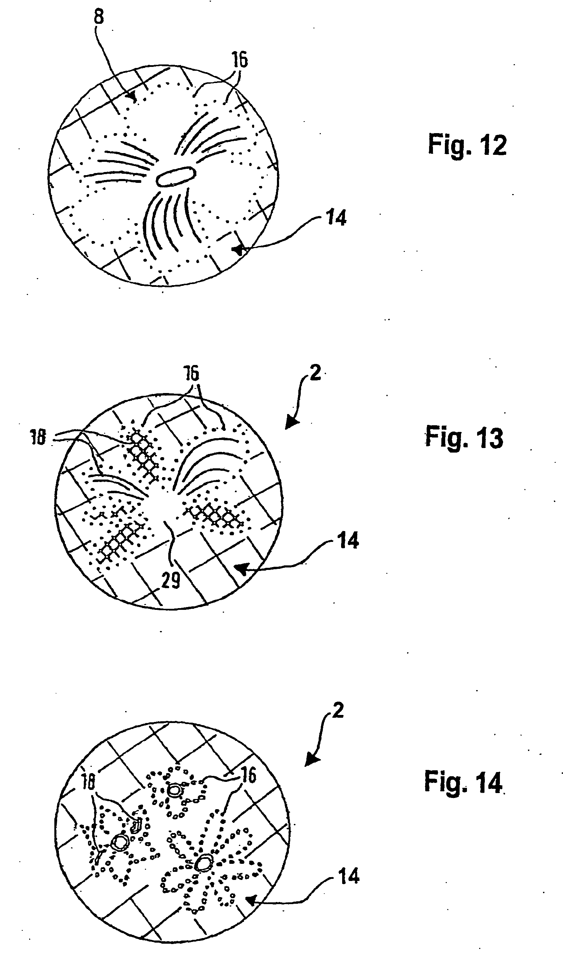

Auch die nachfolgenden Figuren 13 und 14 dienen ausschließlich der detaillierten Darstellung von weiteren Ausführungsformen der ersten Prägestruktur 8. Erfindungsgemäß sind auch die Wattepads mit den ersten Prägestrukturen nach Figuren 13 und 14 von einem äußeren Bereich 10 umfassend eine zweite Prägestruktur 14 vollumfänglich umgeben.

Bei dem Wattepad nach Figur 12 setzt sich die erste Prägestruktur 8 aus Prägepunkten (16) und Prägelinien (18) zusammen, wobei die Prägepunkte (16) in ihrer Ausführungsform als Durchprägung gestaltet sind. Bei dem Wattepad nach Figur 13 zeigt die erste Prägestruktur 8 mit Prägepunkten (16) und Prägelinien (18) einen in Umfangsrichtung offenen Abschnitt (29).

Bei dem Wattepad nach Figur 14 setzt sich die erste Prägestruktur 8 aus 3 geschlossenen Strukturen, jeweils in Form einer Blumenstruktur, zusammen. Die einzelnen Strukturen umfassen Prägepunkte (16) und Prägelinien (18).

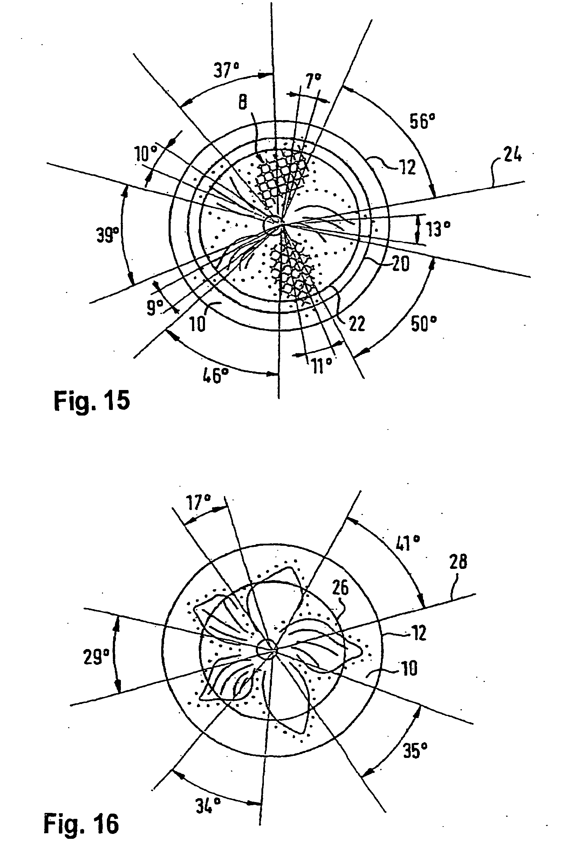

Die Darstellungen in den Figuren 15 bis 18 dienen der Veranschaulichung der Berechnung der Umfangswinkel, innerhalb deren die radiale Erstreckung des äußeren Bereiches 10 vom Rand nach innen wenigstens 5 mm bzw. wenigstens 8 mm bzw. wenigstens 10 mm beträgt. Aus Gründen der Übersichtlichkeit ist in den Figuren 15 bis 18 auf die Darstellung der erfindungsgemäßen zweiten Prägestruktur 14 innerhalb des äußeren Bereiches 10 verzichtet worden. Bei der Darstellung nach Figur 15 des Wattepads mit einer ersten Prägestruktur 8 nach Figur 7 ist eine erste virtuelle kreisförmige Linie 20 in einem Abstand von 5 mm vom Rand 12 eingezeichnet und eine zweite virtuelle kreisförmige Linie 22 im Abstand von 8 mm. Sodann wurden virtuelle radiale Linien 24 vom Mittelpunkt des Wattepads nach außen gezogen, und zwar durch jeweilige Schnittpunkte der Prägestruktur mit der ersten und zweiten Linie 20 und 22. Sofern kein wirklicher Schnittpunkt der Prägestruktur durch ein Prägeelement, wie beispielsweise Prägepunkt 16 oder Prägelinie 18, und der virtuellen kreisförmigen Linie gegeben ist, so wird eine kürzeste Verbindung der nächstliegenden Prägeelemente angedacht und dann der Schnittpunkt dieser virtuellen Verbindungslinie mit der virtuellen kreisförmigen Linie ermittelt. Durch Bestimmung der Winkel der durch die Linien 24 gebildeten Kreissegmente und durch entsprechende Aufsummierung der einzelnen Winkel der Kreissegmente lässt sich in Bezug auf die erste Prägestruktur 8 der gesamte Umfangswinkel ermitteln, innerhalb dessen die radiale Erstreckung des äußeren Bereichs 10 vom Rand 12 nach innen wenigstens 5 mm beziehungsweise wenigstens 8 mm beträgt. Bei der Darstellung nach Figur 13 überfängt der äußere Bereich 10 mit einer radialen Erstreckung von mindestens 5 mm vom Rand 12 ca. 310° und derjenige mit einer Erstreckung von wenigstens 8 mm umfängt ca. 228°.

In der Darstellung nach Figur 16 des Wattepads mit der ersten Prägestruktur nach Figur 6 ist eine virtuelle kreisförmige Linie 26 in einem Abstand von 10 mm vom Rand 12 des Wattepads zusammen mit virtuellen radialen Linien 28 eingezeichnet. Der gesamte Umfangswinkel desjenigen äußeren Bereichs 10, der eine radiale Erstreckung vom Rand von wenigstens 10 mm aufweist beträgt ca. 156°.

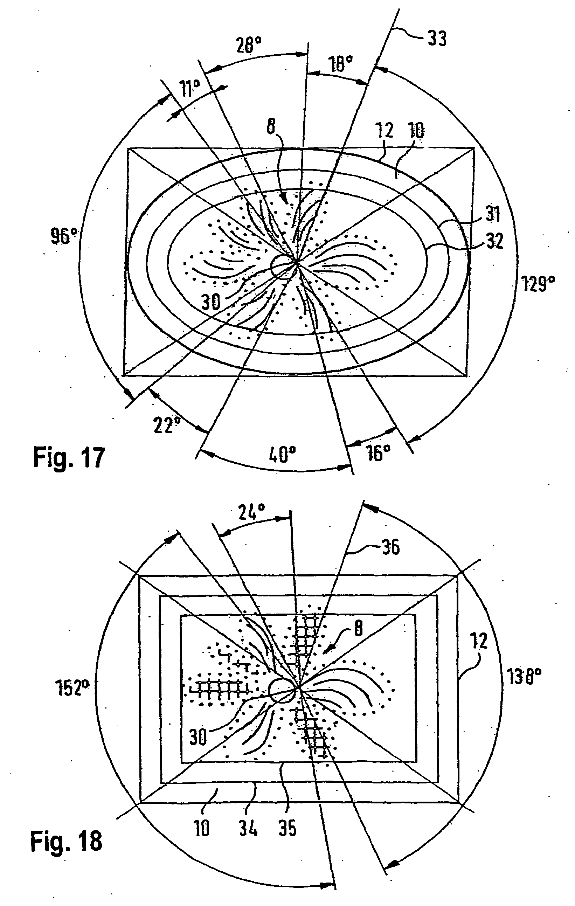

In der Darstellung von Figur 17 bzw. Figur 18 handelt es sich um ein ovales bzw. rechteckiges Wattepad mit jeweils einer ersten Prägestruktur 8.

Figur 17 verdeutlicht für ein ovales Wattepad die Bestimmung der Größe der Winkel der Kreissegmente, innerhalb derer die radiale Erstreckung des äußeren Bereiches 10 vom Rand 12 nach innen wenigstens 5 mm bzw. wenigstens 10 mm beträgt.

Im Wattepad nach Figur 17 sind hierzu zwei virtuelle ovale Linien 31 bzw. 32 im Abstand von 5 mm bzw. 10 mm innerhalb des Randes 12 eingezeichnet. Dann wird das Symmetriezentrum 30 des Wattepads ermittelt. Ausgehend von diesem Symmetriezentrum werden wiederum virtuelle radiale Linien 33 nach außen gezogen, und zwar durch die Schnittpunkte der ersten Prägestruktur 8 mit den virtuellen Linien 31 bzw. 32, wobei virtuelle Kreissegmente entstehen.

Bei Wattepads mit nicht kreisförmigem Umfang, wie beispielsweise nach Figur 15, wird das Symmetriezentrum 30 des Wattepads als Bezugspunkt herangezogen, so dass für die Definition der Abschnitte auch bei diesen Wattepads sinngemäß dieser Anmeldung die Angaben in Form von Gradmaßen (°) gemacht werden.

Bei der Darstellung nach Figur 18 ist der äußere Bereich 10 mit einer radialen Erstreckung von mindestens 5 mm vollumfänglich und derjenige des ersten Bereichs 10 mit einer Erstreckung von wenigstens 10 mm umfängt ca. 293°.

Figur 18 verdeutlicht bei einem rechteckigen Wattepad die Bestimmung der Größe der Winkel der Kreissegmente, innerhalb derer die radiale Erstreckung des äußeren Bereiches 10 vom Rand 12 nach innen wenigstens 5 mm bzw. wenigstens 10 mm beträgt.

In das rechteckige Wattepad nach Figur 16 sind virtuelle, rechteckig verlaufende Linien 34 bzw. 35 im Abstand von 5 mm bzw. 10 mm innerhalb von Rand 12 eingezeichnet. Ausgehend vom Symmetriezentrum 30 werden wiederum virtuelle radiale Linien 36 zu den Schnittpunkten zwischen ersten Prägestruktur 8 und den rechteckig verlaufenden Linien 34 und 35 gezogen. Wiederum lässt sich durch Bestimmung der Winkel der durch die Linien 36 gebildeten Kreissegmente und durch entsprechende Aufsummierung der einzelnen Winkel der Kreissegmente in Bezug auf die erste Prägestruktur 8 der gesamte Umfangswinkel ermitteln, innerhalb deren die radiale Erstreckung des äußeren Bereichs 10 vom Rand 12 nach innen wenigstens 5 mm bzw. wenigstens 10 mm beträgt. Der erste Bereich 10 mit einer Erstreckung von wenigstens 10 mm umfängt hierbei ca. 314°.

**Patentansprüche**

1. Kosmetisches Wattepad (2) mit wenigstens einer Faservlieslage und einer ersten und einer zweiten Seite (4, 6) und mit einer ersten Prägestruktur (8) und einer davon verschiedenen zweiten Prägestruktur (14) auf wenigstens einer Seite (4, 6), gegebenenfalls mit einer zusätzlichen durch Wasserstrahlvernadelung gebildeten Feinrillenstruktur auf einer oder beiden Oberflächen, **dadurch gekennzeich-**

**net, dass** die erste Prägestruktur (8) vollumfänglich von einem äußeren Bereich (10) mit einer von der ersten Prägestruktur verschiedenen zweiten Prägestruktur (14) umgeben ist und dass die zweite Prägestruktur (14) sich nicht in die erste Prägestruktur (8) hinein erstreckt.

2. Kosmetisches Wattepad nach Anspruch 1, **dadurch gekennzeichnet, dass** die radiale Erstreckung des äußeren Bereiches (10) ausgehend vom Rand (12) nach radial innen zumindest abschnittsweise wenigstens 5 mm beträgt.

3. Kosmetisches Wattepad nach Anspruch 1, **dadurch gekennzeichnet, dass** der äußere Bereich (10) über einen Abschnitt von wenigstens 180° in Umfangsrichtung, insbesondere von wenigstens 270°, insbesondere von wenigstens 280°, insbesondere von wenigstens 290°, insbesondere von wenigstens 300°, insbesondere von wenigstens 310° die radiale Erstreckung vom Rand nach innen von wenigstens 5 mm aufweist.

4. Kosmetisches Wattepad nach Anspruch 1 oder 2, **dadurch gekennzeichnet, dass** die radiale Erstreckung des äußeren Bereiches (10) ausgehend vom Rand (12) nach innen über die gesamte Umfangsrichtung wenigstens 3 mm, insbesondere wenigstens 4 mm und weiter insbesondere wenigstens 5 mm beträgt.

5. Kosmetisches Wattepad nach einem oder mehreren der vorstehenden Ansprüche, **dadurch gekennzeichnet, dass** die radiale Erstreckung des äußeren Bereiches (10) ausgehend vom Rand (12) nach innen zumindest abschnittsweise wenigstens 8 mm beträgt.

6. Kosmetisches Wattepad nach Anspruch 5, **dadurch gekennzeichnet, dass** der äußere Bereich (10) über einen Abschnitt von wenigstens 180° in Umfangsrichtung, insbesondere von wenigstens 190°, insbesondere von wenigstens 200° eine radiale Erstreckung vom Rand nach innen von wenigstens 8 mm aufweist.

7. Kosmetisches Wattepad nach einem oder mehreren der vorstehenden Ansprüche, **dadurch gekennzeichnet, dass** die radiale Erstreckung des äußeren Bereiches (10) ausgehend vom Rand (12) nach innen zumindest abschnittsweise wenigstens 10 mm beiträgt.

8. Kosmetisches Wattepad nach Anspruch 6, **dadurch gekennzeichnet, dass** der äußere Bereich (10) über einen Abschnitt von wenigstens 120° in Umfangsrichtung, insbesondere von wenigstens 150°, insbesondere von wenigstens 160° eine radiale Erstreckung vom Rand (12) nach innen von wenigstens 10 mm aufweist.

9. Kosmetisches Wattepad nach einem oder mehreren der vorstehenden Ansprüche, **dadurch gekennzeichnet, dass** die erste Prägestruktur (8) eine Fläche der Prägelinien (18) oder Prägestellen (16) von 1 - 8 %, insbesondere von 2 - 7 %, und weiter insbesondere von 3 - 6 % der Oberfläche der betreffenden Seite (4, 6) umfasst.

10. Kosmetisches Wattepad nach einem oder mehreren der vorstehenden Ansprüche, **dadurch gekennzeichnet, dass** der äußere Bereich (10) einen Flächenanteil von wenigstens 30 %, insbesondere von wenigstens 35 %, insbesondere von wenigstens 40 %, insbesondere von wenigstens 45 % und weiter insbesondere von wenigstens 48 %, insbesondere von wenigstens 50 % und weiter insbesondere von 50 % bis 65 % der Oberfläche der betreffenden Seite (4, 6) umfasst.

11. Kosmetisches Wattepad nach einem oder mehreren der vorstehenden Ansprüche, **dadurch gekennzeichnet, dass** die erste Prägestruktur (8) und/oder zweite Prägestruktur (14) eine Breite der Prägestellen (18, 16) in der Padebene von 0,2 - 0,7 mm aufweist.

12. Kosmetisches Wattepad nach einem oder mehreren der vorstehenden Ansprüche, **dadurch gekennzeichnet, dass** die erste Prägestruktur (8) eine visuell oder taktil wahrnehmbare Information vermittelt, insbesondere ein Dekorationselement, weiter insbesondere einen Schriftzug darstellt.

13. Kosmetisches Wattepad nach einem oder mehreren der vorstehenden Ansprüche, **dadurch gekennzeichnet, dass** die zweite Prägestruktur 14 einen Prägeflächenanteil von 3% bis 11%, insbesondere von 5% bis 10% und weiter insbesondere von 7 % bis 9,5% der Oberfläche einer betreffenden Oberseite (4, 6) des Wattepads umfasst.

14. Kosmetisches Wattepad nach einem oder mehreren der vorstehenden Ansprüche, **dadurch gekennzeichnet, dass** die erste Prägestruktur (8) einen Anteil an verprägter Fläche innerhalb der von erster Prägestruktur eingenommenen Fläche von 5,5 % bis 14,0 %, bevorzugt von 7,5 % bis 12,5 %, insbesondere bevorzugt 9,5% bis 11,5 % aufweist.

15. Kosmetisches Wattepad nach einem oder mehreren der vorstehenden Ansprüche, **dadurch gekennzeichnet, dass** die zweite Prägestruktur (14) einen Anteil an verprägter Fläche innerhalb der von der zweiten Prägestruktur eingenommenen Fläche von wenigstens 12 %, bevorzugt von wenigstens 14 %, weiter bevorzugt von wenigstens 16%, insbesondere bevorzugt von wenigstens 18% bis höchstens 22%, weiterhin bevorzugt von 19 % bis 21% aufweist.

16. Kosmetisches Wattepad nach einem oder mehreren der vorstehenden Ansprüche, **dadurch gekennzeichnet, dass** es eine Reißfestigkeit im trockenen Zustand in Längsrichtung und in Querrichtung von 3-20 N/25 mm aufweist.

17. Kosmetisches Wattepad nach einem oder mehreren der vorstehenden Ansprüche, **dadurch gekennzeichnet, dass** es ein Flächengewicht von 70 - 350 g/m$^2$, insbesondere von 100 - 300 g/m$^2$, weiter insbesondere von 150 - 250 g/m$^2$ aufweist.

18. Kosmetisches Wattepad nach einem oder mehreren der vorstehenden Ansprüche, **dadurch gekennzeichnet, dass** es aus 100 % Baumwollfasern gebildet ist.

19. Kosmetisches Wattepad nach einem oder mehreren der vorstehenden Ansprüche, **dadurch gekennzeichnet, dass** es neben cellulosischen Fasern Zusätze von wärmeschmelzbaren thermoplastischen Fasern, insbesondere Mehrkomponentenfasern, umfasst.

20. Kosmetisches Wattepad nach einem oder mehreren der vorstehenden Ansprüche, **dadurch gekennzeichnet, dass** es Mikrofasern, insbesondere Mikrostapelfasern, von 40-90 Gew-%, bevorzugt von 15-85 Gew-%, weiter bevorzugt von 15-65 Gew-%, insbesondere bevorzugt von 20-30 Gew-% in der Faservlieslage aufweist.

**Claims**

1. Cosmetic cotton wool pad (2) with at least one fibre web layer and a first and a second side (4, 6) and with a first embossed structure (8) and a second embossed structure (14) different therefrom on at least one side (4, 6), optionally with an additional fine-groove structure formed by water-jet needling on one or both surfaces, **characterized in that** the first embossed structure (8) is completely surrounded by an outer region (10) having a second embossed structure (14) different from the first embossed structure, and that the second embossed structure (14) does not extend into the first embossed structure (8).

2. Cosmetic cotton wool pad according to Claim 1, **characterized in that** the radial extension of the outer region (10) starting from the edge (12) radially inwards is at least 5 mm at least in sections.

3. Cosmetic cotton wool pad according to Claim 1, **characterized in that** the outer region (10) has the radial extension from the edge inwards of at least 5 mm over a section of at least 180° in circumferential direction, in particular of at least 270°, in particular of at least 280°, in particular of at least 290°, in particular of at least 300°, in particular of at least 310°.

4. Cosmetic cotton wool pad according to Claim 1 or 2, **characterized in that** the radial extension of the outer region (10) starting from the edge (12) inwards is at least 3 mm, in particular at least 4 mm and further in particular at least 5 mm, over the entire circumferential direction.

5. Cosmetic cotton wool pad according to one or more of the preceding claims, **characterized in that** the radial extension of the outer region (10) starting from the edge (12) inwards is at least 8 mm at least in sections.

6. Cosmetic cotton wool pad according to Claim 5, **characterized in that** the outer region (10) has a radial extension from the edge inwards of at least 8 mm over a section of at least 180° in circumferential direction, in particular of at least 190°, in particular of at least 200°.

7. Cosmetic cotton wool pad according to one or more of the preceding claims, **characterized in that** the radial extension of the outer region (10) starting from the edge (12) inwards is at least 10 mm at least in sections.

8. Cosmetic cotton wool pad according to Claim 6, **characterized in that** the outer region (10) has a radial extension from the edge (12) inwards of at least 10 mm over a section of at least 120° in circumferential direction, in particular of at least 150°, in particular of at least 160°.

9. Cosmetic cotton wool pad according to one or more of the preceding claims, **characterized in that** the first embossed structure (8) includes an area of the embossed lines (18) or embossed points (16) of 1-8%, in particular of 2-7%, and further in particular of 3-6% of the surface of the side (4, 6) in question.

10. Cosmetic cotton wool pad according to one or more of the preceding claims, **characterized in that** the outer region (10) includes an areal fraction of at least 30%, in particular of at least 35%, in particular of at least 40%, in particular of at least 45% and further in particular of at least 48%, in particular of at least 50% and further in particular of 50% to 65%, of the surface of the side (4, 6) in question.

11. Cosmetic cotton wool pad according to one or more of the preceding claims, **characterized in that** the first embossed structure (8) and/or second embossed structure (14) has a width of the embossed points (18, 16) in the pad plane of 0.2-0.7 mm.

12. Cosmetic cotton wool pad according to one or more of the preceding claims, **characterized in that** the first embossed structure (8) imparts visually or tactilely perceptible information, in particular a decorative element, further in particular writing.

13. Cosmetic cotton wool pad according to one or more of the preceding claims, **characterized in that** the second embossed structure 14 includes an embossed areal fraction of 3% to 11 %, in particular of 5% to 10% and further in particular of 7% to 9.5%, of the surface of a top side (4, 6) of the cotton wool pad in question.

14. Cosmetic cotton wool pad according to one or more of the preceding claims, **characterized in that** the first embossed structure (8) has a fraction of embossed area within the area taken up by the first embossed structure of 5.5% to 14.0%, preferably of 7.5% to 12.5%, particularly preferably 9.5% to 11.5%.

15. Cosmetic cotton wool pad according to one or more of the preceding claims, **characterized in that** the second embossed structure (14) has a fraction of embossed area within the area taken up by the second embossed structure of at least 12%, preferably of at least 14%, further preferably of at least 16%, especially preferably of at least 18% to at most 22%, further preferably of 19% to 21%.

16. Cosmetic cotton wool pad according to one or more of the preceding claims, **characterized in that** it has a tear strength in the dry state in longitudinal direction and in transverse direction of 3-20 N/25 mm.

17. Cosmetic cotton wool pad according to one or more of the preceding claims, **characterized in that** it has an areal weight of 70-350 g/m$^2$, in particular of 100-300 g/m$^2$, further in particular of 150-250 g/m$^2$.

18. Cosmetic cotton wool pad according to one or more of the preceding claims, **characterized in that** it is formed from 100% cotton fibres.

19. Cosmetic cotton wool pad according to one or more of the preceding claims, **characterized in that**, besides cellulosic fibres, it comprises additives of heat-meltable thermoplastic fibres, in particular multicomponent fibres.

20. Cosmetic cotton wool pad according to one or more of the preceding claims, **characterized in that** it has microfibers, in particular microstaple fibres, from 40-90% by weight, preferably from 15-85% by weight, further preferably from 15-65% by weight, particularly preferably from 20-30% by weight, in the fibre web layer.

## Revendications

1. Tampon de coton cosmétique (2) comprenant au moins une couche de non-tissé fibreux et une première et une seconde face (4, 6) et une première structure gaufrée (8) et une seconde structure gaufrée (14) différente de celle-ci sur au moins une face (4, 6), éventuellement une structure supplémentaire à côtes fines constituée par aiguilletage au jet d'eau sur une ou deux surfaces, **caractérisé en ce que** la première structure gaufrée (8) est entourée sur toute sa périphérie d'une partie extérieure (10) comportant une seconde structure gaufrée (14) différente de la première structure gaufrée et **en ce que** la seconde structure gaufrée (14) ne s'étend pas dans l'intérieur de la première structure gaufrée (8).

2. Tampon de coton cosmétique selon la revendication 1, **caractérisé en ce que** l'extension radiale de la partie extérieure (10), en partant du bord (12) et radialement vers l'intérieur, s'élève du moins par sections à au moins 5 mm.

3. Tampon de coton cosmétique selon la revendication 1, **caractérisé en ce que** la partie extérieure (10), sur une section d'au moins 180° dans le sens circonférentiel, notamment d'au moins 270°, notamment d'au moins 280°, notamment d'au moins 290°, notamment d'au moins 300°, notamment d'au moins 310°, présente une extension radiale du bord vers l'intérieur d'au moins 5 mm.

4. Tampon de coton cosmétique selon la revendication 1 ou 2, **caractérisé en ce que** l'extension radiale de la partie extérieure (10), en partant du bord (12) vers l'intérieur, sur l'ensemble du sens circonférentiel, s'élève à au moins 3 mm, notamment au moins 4 mm et encore plus notamment au moins 5 mm.

5. Tampon de coton cosmétique selon l'une quelconque ou plusieurs des revendications précédentes, **caractérisé en ce que** l'extension radiale de la partie extérieure (10), en partant du bord (12) vers l'intérieur, s'élève du moins par sections à au moins 8 mm.

6. Tampon de coton cosmétique selon la revendication 5, **caractérisé en ce que** la partie extérieure (10) présente sur une section d'au moins 180° dans le sens circonférentiel, notamment d'au moins 190°, notamment d'au moins 200°, une extension radiale du bord vers l'intérieur d'au moins 8 mm.

7. Tampon de coton cosmétique selon l'une quelconque ou plusieurs des revendications précédentes, **caractérisé en ce que** l'extension radiale de la partie extérieure (10), en partant du bord (12) vers l'intérieur, s'élève du moins par sections à au moins 10 mm.

8. Tampon de coton cosmétique selon la revendication 6, **caractérisé en ce que** la partie extérieure (10) présente sur une section d'au moins 120° dans le sens circonférentiel, notamment d'au moins 150°, notamment d'au moins 160°, une extension radiale du bord (12) vers l'intérieur d'au moins 10 mm.

9. Tampon de coton cosmétique selon l'une quelconque ou plusieurs des revendications précédentes, **caractérisé en ce que** la première structure gaufrée (8) englobe une surface de lignes gaufrées (18) ou de points gaufrés (16) représentant 1 à 8 %, notamment 2 à 7 % et encore plus notamment 3 à 6 % de la surface de la face concernée (4, 6).

10. Tampon de coton cosmétique selon l'une quelconque ou plusieurs des revendications précédentes, **caractérisé en ce que** la partie extérieure (10) englobe une proportion surfacique représentant au moins 30 %, notamment au moins 35 %, notamment au moins 40 %, notamment au moins 45 % et encore plus notamment au moins 48 %, notamment au moins 50 % et encore plus notamment au moins 50 % à 65 % de la surface de la face concernée (4, 6).

11. Tampon de coton cosmétique selon l'une quelconque ou plusieurs des revendications précédentes, **caractérisé en ce que** la première structure gaufrée (8) et/ou seconde structure gaufrée (14) présente une largeur de points gaufrés (18, 16) de 0,2 à 0,7 mm dans le plan du tampon.

12. Tampon de coton cosmétique selon l'une quelconque ou plusieurs des revendications précédentes, **caractérisé en ce que** la première structure gaufrée (8) transmet une information perceptible de manière visuelle ou tactile, représente notamment un élément de décoration, encore plus notamment une inscription.

13. Tampon de coton cosmétique selon l'une quelconque ou plusieurs des revendications précédentes, **caractérisé en ce que** la seconde structure gaufrée (14) comprend une proportion surfacique gaufrée représentant 3 % à 11 %, notamment 5 % à 10 % et encore plus notamment 7 % à 9,5 %, de la surface d'une face supérieure concernée (4, 6) du tampon de coton.

14. Tampon de coton cosmétique selon l'une quelconque ou plusieurs des revendications précédentes, **caractérisé en ce que** la première structure gaufrée (8) présente une proportion de surface gaufrée représentant 5,5 % à 14,0 %, de préférence 7,5 % à 12,5 % et de manière particulièrement préférentielle 9,5 % à 11,5 % de la surface circonscrite par la première structure gaufrée.

15. Tampon de coton cosmétique selon l'une quelconque ou plusieurs des revendications précédentes, **caractérisé en ce que** la seconde structure gaufrée (14) présente une proportion de surface gaufrée représentant au moins 12 %, de préférence au moins 14 %, de manière plus préférentielle au moins 16 %, de manière notamment préférentielle au moins 18 % à 22 % au plus et de manière encore plus préférentielle 19 % à 21 % de la surface circonscrite par la seconde structure gaufrée.

16. Tampon de coton cosmétique selon l'une quelconque ou plusieurs des revendications précédentes, **caractérisé en ce qu'**il présente une résistance à la rupture en état sec de 3 à 20 N/25 mm dans le sens longitudinal et dans le sens transversal.

17. Tampon de coton cosmétique selon l'une quelconque ou plusieurs des revendications précédentes, **caractérisé en ce qu'**il présente un grammage de 70 à 350 g/m$^2$, notamment de 100 à 300 g/m$^2$, encore plus notamment de 150 à 250 g/m$^2$.

18. Tampon de coton cosmétique selon l'une quelconque ou plusieurs des revendications précédentes, **caractérisé en ce qu'**il est composé de fibres 100 % coton.

19. Tampon de coton cosmétique selon l'une quelconque ou plusieurs des revendications précédentes, **caractérisé en ce qu'**il comprend, outre des fibres cellulosiques, des ajouts de fibres thermoplastiques thermofusibles, notamment de fibres multicomposants.

20. Tampon de coton cosmétique selon l'une quelconque ou plusieurs des revendications précédentes, **caractérisé en ce qu'**il présente des microfibres, notamment des microfibres discontinues représentant 40 à 90 % en poids, de préférence 15 à 85 % en poids, de manière plus préférentielle 15 à 65 % en poids, de manière particulièrement préférentielle 20 à 30 % en poids de la couche de non-tissé fibreux.

**Fig. 1**

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

**Fig. 15**

**Fig. 16**

Fig. 17

Fig. 18

Fig. 19a

Fig. 19b

**IN DER BESCHREIBUNG AUFGEFÜHRTE DOKUMENTE**

**In der Beschreibung aufgeführte Patentdokumente**

- EP 1106723 A1
- EP 1310226 A2
- EP 0405043 B1
- WO 03090653 A